(19) Europäisches Patentamt
European Patent Office
Office européen des brevets

(11) Publication number: **0 018 189**
**B1**

(12) **EUROPEAN PATENT SPECIFICATION**

(45) Date of publication of patent specification: **12.09.84**

(51) Int. Cl.$^3$: **A 61 K 39/39,** A 61 K 39/08, A 61 K 39/13

(21) Application number: **80301183.2**

(22) Date of filing: **15.04.80**

(54) **Synthetic adjuvants for stimulating antigenic responses.**

(30) Priority: **18.04.79 CA 325670**

(43) Date of publication of application: **29.10.80 Bulletin 80/22**

(45) Publication of the grant of the patent: **12.09.84 Bulletin 84/37**

(84) Designated Contracting States: **CH DE FR GB IT LI NL SE**

(56) References cited:
**CH-A- 371 456**
**GB-A-1 089 767**
**US-A-3 708 558**

**D. Gall "Immunology" 1966 (11) pp. 369-386**

(73) Proprietor: **CONNAUGHT LABORATORIES LIMITED**
**1755 Steeles Avenue West**
**Willowdale, Ontario, Canada M2N 5T8 (CA)**

(72) Inventor: **Moloney, Peter Joseph**
**50 St. Joseph Street**
**Toronto Ontario (CA)**
Inventor: **Wojcik, George**
**191 St. George Street Apartment 702**
**Toronto Ontario (CA)**

(74) Representative: **Dew, Melvyn John et al**
**Haseltine Lake & Co Hazlitt House 28**
**Southampton Buildings Chancery Lane**
**London WC2A 1AT (GB)**

**EP 0 018 189 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

The present invention is concerned with antigen compositions having improved antigenic capability.

An "adjuvant" is a substance that itself is often biologically inactive but which, in conjunction with a given antigen, enhances its antigenic capabilities. Adjuvants have been used experimentally in animals for studies on the immune system and in vaccines for improvement of immunological responses, as measured by antibody titres. The enhancing effects have, in many instances, been confirmed by application to humans.

One advantage of the use of adjuvants in vaccines lies in the fact that the same degree of antibody response can be achieved with a smaller amount of antigen. This advantage is demonstrated in the case of an antigen which must be used at a dosage where marked primary reactivity is shown in order to induce a suitable antibody level. Adjuvants are particularly useful in cases where the antigen alone does not stimulate high levels of antibody. A main function of an adjuvant is to raise the antibody response to levels which will ensure protection against an infectious disease.

Any material used as an adjuvant in vaccines should be non-toxic, relatively easily metabolized and produce little or no skin reaction at the injection site.

Prior art adjuvants range in type from simple inorganic materials, such as aluminium phosphate, to complex mixtures, such as Freund's adjuvant which is a homogenate of oil, detergent and killed tubercle bacilli.

In CH—A—371456 (Ciba) there are disclosed compounds which are stated to be useful as adjuvants and which are obtained by reacting together an aliphatic acid (or ester forming derivative thereof) with an amine substituted alkyl hydroxylamine.

GB—A—1 089 767 discloses adjuvants for vaccines having the general formula

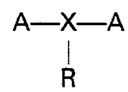

wherein A is an aliphatic radical having from 12 to 22 carbon atoms, X is a nitrogen atom or a guanidine residue, and R is hydrogen or a lower alkyl group. These compounds are organic amines and may be either in the free base form or in the form of a quaternised salt.

In accordance with US—A—3 708 558, an adjuvant is produced by reacting a long chain ether alcohol of the formula $HO—CH_2—CH_2—CH_2—O—C_{18}H_{37}$ with a brominated phosphoric acid ester to produce an ester of the formula

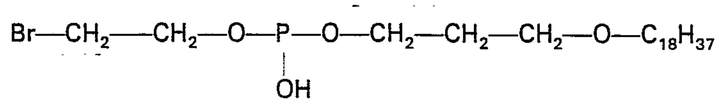

and then reacting this ester with trimethylamine to obtain the desired adjuvant having the formula

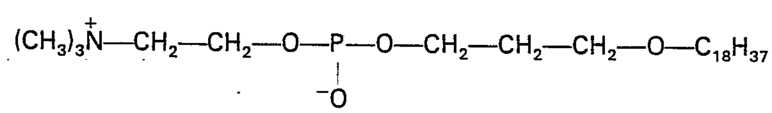

D. Gall in an article entitled "The Adjuvant Activity of Aliphatic Nitrogenous Bases" in Immunology, 1966, 11, p. 369—386 discloses that a number of aliphatic nitrogenous bases including amines, quaternary ammonium compounds, guanidines, benzamidines and thiouroniums exhibit high adjuvant activity in respect of diphtheria toxoid in guinea pigs but that such adjuvants tend to be haemolytic.

The present invention provides a novel antigen composition containing certain compounds which have not heretofore been used as adjuvants. These compounds meet the above-noted requirements for vaccine use and result in a higher antigenic capability than prior known adjuvants.

According to the present invention there is provided an antigen composition comprising at least one bacterial or viral antigenic species and at least one adjuvant in an amount effective to enhance the antigenic response of the antigenic species, characterized in that the adjuvant is an amino acid ester of a long chain alcohol containing 10 to 22 carbon atoms or a salt of such an amino acid ester.

In the case where the adjuvant is an amino acid ester salt, it may be for example the hydrochloride salt. The esters are generally insoluble or only slightly soluble in aqueous solutions at neutral pH.

The esters are formed from a long chain alcohol and an amino acid.

The long chain alcohol may be any alkanol of the general formula R—OH, where R is an alkyl group containing 10 to 22, and preferably 12 to 22, carbon atoms. The alkyl group will usually be an *n*-alkyl group, and examples of suitable alcohols include *n*-decanol, *n*-dodecanol, myristyl alcohol, cetyl alcohol and n-octadecanol.

The amino acid may be any amino acid of the formula:

$$R^1\text{—(alkylene)—}COOH$$
$$|$$
$$NH_2$$

where $R^1$ is hydrogen or an organic group. The alkylene group will usually be a (CH) group, i.e., the amino acid will usually be an $\alpha$-amino acid. Examples of amino acids which may be used include leucine, tyrosine and tryptophane.

A particularly preferred amino acid ester is the ester of octadecanol and tyrosine, which has been found to be particularly effective as an adjuvant. Octadecyl tyrosine may be used in the form of its hydrochloride salt.

The esters used in this invention may be formed by any convenient ester-forming process. For example, the alcohol and the amino acid may be heated together under reflux while hydrogen chloride gas is bubbled through the mixture, followed by purification and isolation of the ester as the hydrochloride salt.

The amino acid ester is added to an antigen, which is bacterial or viral, to provide the antigen compositions of the invention. The use of octadecyl tyrosine as adjuvant with tetranus toxoid and poliomyelitis vaccine has been found to be particularly effective.

In use of the antigen composition of the invention to administer the antigenic species to a warm-blooded animal, a dose of the composition is injected into the animal to enhance the antigenic response of the antigenic species. Subsequently the animal is injected with a dose of the antigenic species alone.

The quantity of adjuvant used depends on the antigen with which it is used and the antigen dosage to be applied. Usually the quality of amino acid ester added is within the range conventionally used for adjuvants. For example, in the case of tetanus toxoid, where the normal dose is about 30 Lf/ml, quantities of adjuvant of about 0.3 to about 2 mg/ml may be used.

The invention is illustrated further by the following Examples:

Example 1

This Example illustrates the preparation of octadecyl tyrosine hydrochloride.

Octadecanol (8 g) and 1-tyrosine (3 g) were placed in a 250 ml round-bottomed flask together with a magnetic stirring bar. The flask was fitted with a reflux condenser and heated to about 120°C for 3 hours. During this time the mixture was stirred and dry hydrochloric acid gas continuously bubbled through at a slow rate.

After cooling the mixture, ethyl ether (50 ml) was added. On standing, the mixture formed a slurry which was centrifuged. The precipitate was washed three times with ether to remove octadecanol. The ether-washed precipitate consisted of unreacted tyrosine and octadecyl tyrosine hydrochloride.

The washed precipitate was suspended in water and the pH adjusted to 8.0 to produce the free base. After drying, the precipitate was extracted with ethyl to dissolve the octadecyl tyrosine free base. Octadecyl tyrosine hydrochloride was obtained by saturating the ether solution with dry hydrogen chloride gas. The yield was 1.25 g of the hydrochloride (yield 16%). Analysis for nitrogen gave 2.96% (calculated 2.98%) giving an estimated purity equivalent to 99%.

Example 2

This Example sets forth procedures for the preparation of suspensions of octadecyl tyrosine and antigen.

Procedure A:

Tetanus toxoid solution (10 ml, 30 Lf/ml, pH 7.0) was placed in a container with a magnetic stirring bar. An ether solution of octadecyl tyrosine (0.3 ml, 30 mg/ml) was added, the mixture stirred rapidly and the ether removed under vacuum. The resulting cloudy solution was stirred at room temperature for 24 hours. The supernatant, after centrifuging, contained no detectable tetanus toxoid.

Procedure B:

To tetanus toxoid solution (10 ml, 30 Lf/ml, pH 7.0) was added dry octadecyl tyrosine base (10 mg) and the mixture stirred for 24 hours at room temperature. After this time no detectable tetanus toxoid was found in the supernatant.

Octadecyl tyrosine base dissolved in ether can be sterilized by filtration so that the procedures can be carried out aseptically.

Example 3

Three groups of seven guinea pigs were injected with a dose of an antigen composition;

(1) tetanus toxoid adsorbed on octadecyl tyrosine,

(2) tetanus toxoid adsorbed on aluminum phosphate, and

(3) plain tetanus toxoid, respectively.

After four weeks the animals were bled and the antibody titres determined. All three groups were then given a second dose of unadjuvanted tetanus toxoid. Two and one half weeks later they were bled and the antibody titres measured. The results are given in the following Table I:

TABLE I

| Group | 1st Treatment | 2nd Treatment | Antibody Units/ml 4 Weeks | Antibody Units/ml $6\frac{1}{2}$ weeks |
|---|---|---|---|---|
| 1 | 30 Lf Tetanus Toxoid +1 mg/ml Octadecyl Tyrosine — 1 ml/ subcutaneous | 5 Lf Toxoid 0.5 ml subcutaneous | $2.54 \pm 0.4$ (7)[a] | $18 \pm 2.7$ (6) |
| 2 | 30 Lf Tetanus Toxoid + 3 mg/ml $AlPO_4$ — 1 ml/subcutaneous | 5 Lf Toxoid 0.5 ml subcutaneous | $2.35 \pm 0.3$ (7) | $9.7 \pm 1.1$ (6) |
| 3 | 30 Lf Tetanus Toxoid 1 ml subcutaneous | 5 Lf Toxoid 0.5 ml subcutaneous | $1.39 \pm 0.2$ (7) | $7.2 \pm 1.2$ (6) |

(a) The bracketed figures show the number of animals.

Note: After secondary stimulus (six and one half weeks after first injection) octadecyl tyrosine-tetanus toxoid gave a significantly greater antibody response than did tetanus toxoid without adjuvant ($0.01 > p > 0.0001$) and a better response than $AlPO_4$ tetanus toxoid ($0.05 > p > 0.01$)

It can be seen from the results of Table I that after four weeks there is very little difference in the titres of the three groups. However, after the second injection there is a very significant difference in the antibody titres in the group receiving their primary stimulus with toxoid containing the octadecyl tyrosine. The footnote to Table I indicates the degrees of significance.

Example 4

Six guinea pigs which had been sensitized to tetanus toxoid were tested for local skin reactions. Three tetanus toxoids were used for the test: unaltered toxoid, heptadecylamine toxoid and octadecyl tyrosine toxide. Animals were given 2 Lf in 0.1 ml and the diameters of the reactions read 20 hours after the injections. The results are shown in the following Table II:

TABLE II

| | Material | Dose | Diameter in mm |
|---|---|---|---|
| 1. | Unaltered Tetanus Toxoid | 2 Lf/0.1 ml | $17 \pm 1.47$ (6) |
| 2. | Heptadecylamine Tetanus Toxoid | 2 Lf/0.1 ml | $11 \pm 0.97$ (6) |
| 3. | Octadecyl Tyrosine Toxoid | 2 Lf/0.1 ml | $8.3 \pm 0.67$ (6) |

The diameters obtained for the octadecyl tyrosine toxoid reported in the above Table II were statistically smaller than those obtained for the other toxoids.

Example 5

The animals remaining from Example 3 were examined for evidence of reaction at the site of the subcutaneous injections. With the guinea pigs that had received toxoid plus aluminum phosphate there were distinctly palpable nodules of diameter about 8 mm. With the guinea pigs receiving octadecyl tyrosine very small nodules of diameter 2 to 3 mm only were detected in some, but not all, guinea pigs. It should be noted that while the aluminum phosphate was added at a concentration of 3 mg/ml and the octadecyl tyrosine contained only 1 mg/ml, the octadecyl tyrosine toxoid was a better adjuvant at this level than the aluminum phosphate.

Example 6

Three different preparations namely, plain unadsorbed poliomyelitis vaccines (Types 1, 2 and 3), the same poliomyelitis vaccine containing aluminum phosphate as adjuvant and poliomyelitis vaccine containing octadecyl tyrosine were prepared. Each preparation was injected into groups of 8 guinea pigs each as undiluted vaccine and at 1/10 and 1/100 dilutions. Four weeks after injection the animals were bled, antibody levels were measured and a secondary stimulus of plain poliomyelitis vaccine

injected. After a further three weeks the animals were bled and antibody levels determined. The results are shown in the following Table III:

TABLE III

| Preparation | Potency Relative to Unadsorbed Vaccine(1) Type 1 | Type 2 | Type 3 |
|---|---|---|---|
| Poliomyelitis Vaccine with Octadecyl Tyrosine | 13.8 | 3.24 | 8.51 |
| Poliomyelitis Vaccine with Aluminum Phosphate | 2.63 | 1.66 | 6.17 |

Note: (1) Determined by comparison of $ED_{50}$

It is evident from the results of the above Table III that the vaccine containing octadecyl tyrosine gave higher antibody response than either of the other two preparations. If the results are assessed in terms of relative potency of the vaccines, the octadecyl tyrosine is 13.8, 3.2 and 8.5 times more potent than the plain vaccine for types 1, 2 and 3 respectively. It is more potent than the vaccine containing aluminum phosphate by factors of 5.2, 1.9 and 1.4 for the three virus types.

These results imply that a considerable saving of vaccine could be realized using the octadecyl tyrosine adjuvanted material without loss of efficacy.

In summary of this disclosure, the present invention provides a new adjuvant for antigenic compositions which is more effective than conventional adjuvants.

**Claims**

1. An antigen composition comprising at least one bacterial or viral antigenic species and at least one adjuvant in an amount effective to enhance the antigenic response of the antigenic species, characterised in that the adjuvant is an amino acid ester of a long chain alcohol containing 10 to 22 carbon atoms or a salt of such an amino acid ester.

2. A composition as claimed in claim 1, characterised in that the ester is derived from an $\alpha$-amino acid.

3. A composition as claimed in claim 1 or 2, characterised in that the ester is octadecyl tyrosine.

4. A composition as claimed in any one of the preceding claims wherein the salt is a hydrochloride salt.

5. A composition as claimed in any one of the preceding claims wherein the antigenic species is tetanus toxoid.

6. A composition as claimed in any one of Claims 1 to 4 wherein the antigenic species is poliomyelitis vaccine.

**Revendications**

1. Une composition antigène comprenant au moins une espèce antigène virale ou bactérienne et au moins un adjuvant en une quantité efficace pour améliorer la réponse antigène des espèces d'antigène, caractérisée en ce que l'adjuvant est un ester d'amino acide d'un alcool à longue chaîne contenant 10 à 22 atomes de carbone ou un sel d'un tel ester d'amino acide.

2. Une composition telle que revendiquée dans la revendication 1, caractérisée en ce que l'ester dérive d'un alpha-amino acide.

3. Une composition telle que revendiquée dans la revendication 1 ou 2, caractérisée en ce que l'ester est l'octadécyl tyrosine.

4. Une composition telle que revendiquée dans l'une quelconque des revendications précédentes, dans laquelle le sel est un chlorhydrate.

5. Une composition telle que revendiquée dans l'une quelconque des revendications précédentes, dans laquelle les espèces antigènes consistent en toxoïde de tétanos.

6. Une composition telle que revendiquée dans l'une quelconque des revendications 1 à 4, dans laquelle les espèces antigènes consistent en un vaccin poliomyélitique.

**Patentansprüche**

1. Antigenhaltige Zusammensetzung mit wenigstens einer antigenischen Art aus Bakterien oder Viren und wenigstens einem zusätzlichen Hilfsmittel (adjuvant) in einer Menge, die eine Steigerung der antigenischen Reaktion des Antigens bewirkt, dadurch gekennzeichnet, daß das wirkungssteigernde Mittel ein Ester eine Aminosäure mit einem langkettigen Alkohol mit 10 bis 22 C-Atomen oder dessen Salz ist.

2. Zusammensetzung nach Patentanspruch 1, dadurch gekennzeichnet, daß der Ester von einer $\alpha$-Aminosäure abgeleitet ist.

3. Zusammensetzung nach Patentanspruch 1 oder 2, dadurch gekennzeichnet, daß der Ester Octadecyltyrosin ist.

4. Zusammensetzung nach Patentanspruch 1—3, dadurch gekennzeichnet, daß als Salz das Hydrochloridsalz vorliegt.

5. Zusammensetzung nach einem der vorhergehenden Patentansprüche, dadurch gekennzeichnet, daß die Antigen-Art das Tetanustoxoid ist.

6. Zusammensetzung nach Patentanspruch 1—4, dadurch gekennzeichnet, daß die Antigenart Poliovakzine ist.